# EUROPÄISCHE PATENTSCHRIFT

(11) **EP 3 191 078 B1**
(45) Veröffentlichungstag und Bekanntmachung des Hinweises auf die Patenterteilung: **20.03.2019**
(21) Anmeldenummer: 15734103.3
(22) Anmeldetag: 26.06.2015
(51) Int. Cl.: A61K 8/64, A61Q 15/00, A61K 8/9783

(54) **SCHWEISSHEMMENDE KOSMETISCHE MITTEL MIT PROTEINEN AUS MALVENGEWÄCHSEN DER GATTUNG ADANSONIA, WELCHE KEINE HALOGENIDE UND/ODER HYDROXYHALOGENIDE VON ALUMINIUM UND/ODER ZIRCONIUM ENTHALTEN**
SWEAT-INHIBITING COSMETICS WITH PROTEINS FROM MALVAE OF GENUS ADANSONIA, AND WHICH DO NOT CONTAIN ALUMINIUM AND/OR ZIRCONIUM HALOGENIDES AND/OR HYDROXYHALOGNIDES
COMPOSITIONS COSMETIQUES COMPRENANT DES PROTEINES DE MALVAE DU GENRE ADANSONIA, ET QUI NE COMPRENNENT PAS DES HALOGÉNURES ET/OU HYDROXYHALOGÉNURES D'ALUMINIUM ET/OU DE ZIRCONIUM

(30) Priorität: 26.08.2014 DE 102014216913
(43) Veröffentlichungstag der Anmeldung: 19.07.2017
(73) Patentinhaber: Henkel AG & Co. KGaA, 40589 Düsseldorf (DE)
(72) Erfinder: BANOWSKI, Bernhard, 40597 Düsseldorf (DE); EVERS, Stefan, 42781 Haan (DE)
(86) Internationale Anmeldenummer: PCT/EP2015/064557
(87) Internationale Veröffentlichungsnummer: WO 2016/030048

(56) Entgegenhaltungen:
- EP-A1- 2 143 418
- EP-A2- 1 813 310
- WO-A1-94/24993
- WO-A1-2007/104454
- WO-A1-2010/003861
- WO-A1-2010/070143
- FR-A1- 2 758 457
- DATABASE GNPD [Online] MINTEL; 1. Mai 2013 (2013-05-01), "Deo Roll On", XP002744350, Database accession no. 2076617

## Beschreibung

Die vorliegende Erfindung betrifft ein schweißhemmendes kosmetisches Mittel, enthaltend a) mindestens einen Stoff, ausgewählt aus der Gruppe von kosmetischen Ölen, welche bei 20 °C und 1.013 hPa flüssig sind, Riechstoffen und Wachsen, b) Treibmittel in einer Gesamtmenge von 0 bis 99 Gew.-%, bezogen auf das Gesamtgewicht des schweißhemmenden kosmetischen Mittels, und c) mindestens ein aus den Samen der Gattung Adansonia isoliertes Protein, in einer Gesamtmenge von 2 bis 40 Gew.-%, bezogen auf das Gesamtgewicht des schweißhemmenden kosmetischen Mittels, wobei das Protein hydrolysiert ist, wobei das schweißhemmende kosmetische Mittel keine Halogenide und/oder Hydroxyhalogenide von Aluminium und/oder Zirconium enthält.

Weiterhin betrifft die vorliegende Erfindung eine Verpackungseinheit (Kit-of-parts) enthaltend ein erfindungsgemäßes kosmetisches Mittel sowie ein kosmetisches Mittel mit mindestens einem schweißhemmenden Wirkstoff.
Darüber hinaus betrifft die vorliegende Erfindung die Verwendung von speziellen Proteinen aus Malvengewächsen der Gattung *Adansonia* zur zumindest teilweisen Beeinflussung der Schweißdrüse(n).

Schließlich betrifft die vorliegende Erfindung ein nicht-therapeutisches kosmetisches Verfahren zur Verhinderung und/oder Reduzierung der Transpiration des Körpers, bei welchem ein erfindungsgemäßes schweißhemmendes kosmetisches Mittel oder eine erfindungsgemäße Verpackungseinheit auf die Haut, insbesondere auf die Haut der Achselhöhlen, aufgetragen wird und für mindestens 1 Stunde, vorzugsweise für mindestens 2 Stunden, bevorzugt für mindestens 4 Stunden, insbesondere für mindestens 6 Stunden, auf der Haut der Achselhöhlen verbleibt.
Das Waschen, Reinigen und Pflegen des eigenen Körpers stellt ein menschliches Grundbedürfnis dar und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs und der Achselnässe. Im Stand der Technik sind zahlreiche spezielle deodorierende oder schweißhemmende Körperpflegemittel bekannt, welche für die Anwendung in Körperregionen mit einer hohen Dichte von Schweißdrüsen, insbesondere im Achselbereich, entwickelt wurden. Diese sind in den unterschiedlichsten Darreichungsformen konfektioniert, beispielsweise als Puder, in Stiftform, als Aerosolspray, Pumpspray, flüssige und gelförmige Roll-on-Applikation, Creme, Gel und als getränkte flexible Substrate (Deotücher).
Kosmetische Antitranspirantien des Standes der Technik enthalten neben mindestens einem Öl oder einem Wachs und einer Riechstoffkomponente bzw. einem Parfüm mindestens eine schweißhemmende Verbindung, insbesondere in Form von Halogeniden und/oder Hydroxyhalogeniden von Aluminium und/oder Zirconium. Diese schweißhemmenden Verbindungen verringern zum einen die Schweißsekretion des Körpers durch eine temporäre Verengung und/oder Verstopfung der Ausführungsgänge der Schweißdrüsen, so dass die Schweißmenge um etwa 20 bis 60 Prozent reduziert werden kann. Zum anderen weisen sie aufgrund ihrer antimikrobiellen Wirkung einen zusätzlichen desodorierenden Effekt auf.

Antitranspirantien sind auch im Stand der Technik bekannt, welche keine Halogenide/Hydroxyhalogenide von Aluminium und Zirkonium enthalten, z.B. WO 2010/003861 A1 und EP 2 143 418 A1.

Halogenide und/oder Hydroxyhalogenide von Aluminium und/oder Zirconium können in Verbindung mit dem sauren pH-Wert dieser Antitranspirantien bei einigen Anwendern zu unangenehmen Hautreaktionen führen. Darüber hinaus kann die Verwendung der vorgenannten schweißhemmenden Verbindungen zu einer Fleckenbildung auf der Kleidung führen.
Es besteht daher ein Bedarf schweißhemmende Halogenide und/oder Hydroxyhalogenide von Aluminium und/oder Zirconium gegen andere schweißhemmende kosmetische Wirkstoffe auszutauschen. Diese schweißhemmenden Wirkstoffe sollen eine gute schweißhemmende Wirkung, eine gute Hautverträglichkeit sowie eine einfache Formulierbarkeit aufweisen. Darüber hinaus sollen diese schweißhemmenden Wirkstoffe keinen negativen Einfluss auf die Lagerstabilität der schweißhemmenden kosmetischen Mittel aufweisen.
Der vorliegenden Erfindung lag die Aufgabe zugrunde, ein schweißhemmendes kosmetisches Mittel bereitzustellen, welches die Nachteile des Standes der Technik vermeidet bzw. zumindest abschwächt und welches eine gute Hautverträglichkeit bei gleichzeitig zuverlässiger Verminderung der Achselnässe besitzt. Darüber hinaus soll das schweißhemmende kosmetische Mittel eine hohe Lagerstabilität aufweisen.

Es wurde nun überraschend gefunden, dass der Einsatz mindestens ein aus den Samen der Gattung Adansonia isolierten Proteins, in einer Gesamtmenge von 2 bis 40 Gew.-%, bezogen auf das Gesamtgewicht des schweißhemmenden kosmetischen Mittels, wobei das Protein hydrolysiert ist, in kosmetischen Mitteln in einer schweißhemmenden Wirkung resultiert, welche mit der schweißhemmenden Wirkung von Formulierungen mit Aluminiumsalzen und/oder Aluminium-Zirkonium-Komplexen nahezu vergleichbar ist. Die schweißhemmende Wirkung erfindungsgemäßer Mittel wird ohne Zusatz von schweißhemmenden Halogeniden und/oder Hydroxyhalogeniden von Aluminium und/oder Zirconium erreicht.

Durch den Einsatz des mindestens einen hydrolysierten Protein aus Samen von Malvengewächsen der Gattung *Adansonia*, die spezielle physikalische Eigenschaften aufweisen (siehe Beispiel 1), in den erfindungsgemäßen schweißhemmenden kosmetischen Mitteln erfolgt - ohne sich auf diese Theorie beschränken zu wollen - eine gezielte Beeinflussung der Schweißdrüse(n). Diese gezielte Beeinflussung der Schweißdrüse(n) kann beispielsweise in einer Gelbildung des mindestens einen Proteins bei pH-Werten, welche ausschließlich innerhalb der Ausführungsgänge der Schweißdrüsen vorliegen, bestehen. Auf diese Art und Weise kann eine effektive Verstopfung der Ausführungsgänge der Schweißdrüsen gewährleistet werden, ohne dass die schweißhemmende Wirkung des erfindungsgemäßen kosmetischen Mittels durch vorzeitige unerwünschte Gelbildung aufgrund des Zusatzes des mindestens einen speziellen Proteins vermindert wird. Weiterhin kann die gezielte die Beeinflussung der Schweißdrüse(n) jedoch auch in einer Störung des Ladungsgleichgewichts innerhalb der Schweißdrüse(n) bestehen, welche zu einer Beeinflussung der Schweißproduktion, insbesondere zu einer Verminderung der Schweißproduktion, führt. Somit wird auch bei Abwesenheit schweißhemmender Halogenide und/oder Hydroxyhalogenide von Aluminium und/oder Zirconium eine effektive Verminderung des Achselschweißes gewährleistet.

Unter dem Begriff "schweißhemmend" wird erfindungsgemäß die Verminderung bzw. Reduzierung der Transpiration der Schweißdrüsen des Körpers verstanden.

Darüber hinaus werden unter dem Begriff "Halogenide und/oder Hydroxyhalogenide von Aluminium und/oder Zirconium" im Rahmen der vorliegenden Erfindung insbesondere Chloride, Bromide und lodide des Aluminiums und des Zirconiums sowie Verbindungen der Formeln Al(OH)_{y}X und Zr(OH)_{z}X verstanden, wobei X in den vorgenannten Formeln für ein Halogenidion steht.

Weiterhin wird unter dem Begriff "kosmetisches Öl" im Sinne der vorliegenden Erfindung ein für die kosmetische Verwendung geeignetes Öl verstanden, welches mit Wasser nicht in allen Mengen mischbar ist. Bei dem erfindungsgemäß verwendeten kosmetischen Öl handelt es sich weder um Riechstoffe, noch um ätherische Öle.

Zudem werden unter dem Begriff "Riechstoffe" im Sinne der vorliegenden Erfindung Substanzen mit einer Molmasse von 74 bis 300 g/mol verstanden, welche mindestens eine osmophore Gruppe im Molekül enthalten und einen Geruch und/oder Geschmack aufweisen, d. h. sie sind in der Lage, die Rezeptoren der Haarzellen des olfaktorischen Systems zu erregen. Osmophore Gruppen sind kovalent an das Molekülgerüst gebundene Gruppen in Form von Hydroxygruppen, Formylgruppen, Oxogruppen, Alkoxycarbonylgruppen, Nitrilgruppen, Nitrogruppen, Azidgruppen etc. In diesem Zusammenhang fallen unter den Begriff "Riechstoffe" im Sinne der vorliegenden Erfindung auch bei 20 °C und 1.013 hPa flüssige Parfümöle, Parfüme, oder Parfümölbestandteile.

Darüber hinaus werden unter dem Begriff "Wachse" im Rahmen der vorliegenden Erfindung Substanzen verstanden, welche bei 20 °C knetbar oder fest bis brüchig hart sind, eine grobe bis feinkristalline Struktur aufweisen und farblich durchscheinend bis opak, aber nicht glasartig sind. Weiterhin schmelzen diese Substanzen über 25 °C ohne Zersetzung, sind wenig oberhalb des Schmelzpunktes leicht flüssig (wenig viskos), weisen eine stark temperaturabhängige Konsistenz und Löslichkeit auf und sind unter leichtem Druck polierbar.

Der Begriff "Proteine" bezeichnet erfindungsgemäß chemische Verbindungen, welche durch Peptid-Bindungen Säureamid-artig verknüpfte Kondensationsprodukte von Aminosäuren bilden. Die Anzahl der Aminosäuren in den Proteinen beträgt bevorzugt 2 bis 1.000, vorzugsweise 2 bis 500, insbesondere 2 bis 60, Aminosäuren. Unter dem Begriff "Proteine" sind erfindungsgemäß auch Hydrolysate von Proteinen zu verstehen, welche Proteinfraktionen mit verschiedenen Aminosäuresequenzen und Molekulargewichten enthalten. Darüber hinaus sind unter diesem Begriff im Rahmen der vorliegenden Erfindung auch Mischungen von in Malvengewächsen der Gattung *Adansonia* vorkommenden Proteinen zu verstehen.

Weiterhin sind unter dem Begriff "Malvengewächse" im Rahmen der vorliegenden Erfindung Pflanzen aus der Ordnung der Malvenartigen (Malvales) innerhalb der bedecktsamigen Pflanzen zu verstehen. Zu den bekanntesten Nutzpflanzen aus der Familie der Malvengewächse zählen beispielsweise der Kakaobaum, die Baumwollpflanze sowie der Affenbrotbaum.

Darüber hinaus wird unter dem Begriff "Veränderung der Lichtabsorption des mindestens einen Proteins" sowohl die positive und negative Veränderung der Lichtdurchlässigkeit der Probenmischung, insbesondere der Proteinlösung, als auch die Absorption von Licht durch das mindestens eine Protein bzw. die Probenmischung verstanden.

Weiterhin wird unter dem Begriff "pH-Wert Änderung" die kontinuierliche Veränderung des pH-Wertes verstanden. Die kontinuierliche Veränderung des pH-Wertes kann beispielsweise durch Titration, bzw. gleichmäßige Zugabe, einer Base oder Säure erreicht werden.

Der Begriff "Probenmischung" bezeichnet erfindungsgemäß eine Mischung aus dem mindestens einen speziellen Protein mit einem Lösungsmittel, insbesondere Wasser, Puffer oder salzhaltigen wässrigen Lösungen.

Zudem sind unter dem Begriff der "Fettsäuren", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff der "Fettalkohole" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Die Angabe Gew.-% bezieht sich vorliegend, sofern nichts anderes angegeben ist, auf das Gesamtgewicht der erfindungsgemäßen schweißhemmenden kosmetischen Mittel.

Als ersten Bestandteil a) enthalten die erfindungsgemäßen kosmetischen Mittel mindestens einen Stoff, welcher aus der Gruppe von kosmetischen Ölen, welche bei 20 °C und 1.013 hPa flüssig sind, Riechstoffen und Wachsen ausgewählt ist.

Im Rahmen der vorliegenden Erfindung ist das bei 20°C und 1.013 hPa flüssige kosmetische Öl ausgewählt aus der Gruppe von (i) flüchtigen cyclischen Siliconölen, insbesondere cylischen und linearen Siliconölen; (ii) flüchtigen Nichtsiliconölen, insbesondere flüssigen Paraffinölen und Isoparaffinölen; (iii) nichtflüchtigen Siliconölen; (iv) nichtflüchtigen Nichtsiliconölen; sowie (v) deren Mischungen.

Der Begriff "flüchtiges Öl" bezeichnet erfindungsgemäß Öle, welche bei 20 °C und einem Umgebungsdruck von 1.013 hPa einen Dampfdruck von 2,66 Pa bis 40.000 Pa (0,02 bis 300 mm Hg), vorzugsweise von 10 bis 12.000 Pa (0,1 bis 90 mm Hg), weiter bevorzugt von 13 bis 3.000 Pa (0,1 bis 23 mm Hg), insbesondere von 15 bis 500 Pa (0,1 bis 4 mm Hg), aufweisen.

Darüber hinaus werden unter dem Begriff "nichtflüchtige Öle" im Sinne der vorliegenden Erfindung Öle verstanden, welche bei 20 °C und einem Umgebungsdruck von 1.013 hPa einen Dampfdruck von weniger als 2,66 Pa (0,02 mm Hg) aufweisen.

Es kann erfindungsgemäß bevorzugt sein, Mischungen von flüchtigen Siliconölen und flüchtigen Nichtsiliconölen in den erfindungsgemäßen schweißhemmenden kosmetischen Mitteln einzusetzen, da hierdurch ein trockeneres Hautgefühl erreicht wird. Weiterhin kann es im Rahmen der vorliegenden Erfindung bevorzugt sein, wenn die schweißhemmenden kosmetischen Mittel ein nichtflüchtiges Siliconöl und/oder ein nichtflüchtiges Nichtsiliconöl enthalten, um unlösliche Bestandteile, wie Talkum oder auf der Haut angetrocknete Inhaltsstoffe, zu maskieren.

Erfindungsgemäß besonders bevorzugt ist der Einsatz von Mischungen von nichtflüchtigen und flüchtigen kosmetischen Ölen, da auf diese Weise Parameter wie Hautgefühl, Sichtbarkeit des Rückstands und Stabilität des erfindungsgemäßen schweißhemmenden kosmetischen Mittels eingestellt und das Mittel somit besser an die Bedürfnisse der Verbraucher angepasst werden kann.

Die im Rahmen der vorliegenden Erfindung einsetzbaren flüchtigen und nichtflüchtigen Siliconöle sowie flüchtigen und nichtflüchtigen Nichtsiliconöle sind beispielsweise in den Offenlegungsschriften DE 10 2010 063 250 A1 und DE 10 2012 222 692 A1 offenbart.

Gemäß einer bevorzugten Ausführungsform der vorliegenden Erfindung ist das bei 20 °C und 1.013 hPa flüssige kosmetische Öl in einer Gesamtmenge von 0,02 bis 98 Gew.-%, vorzugsweise von 2 bis 85 Gew.-%, bevorzugt von 4 bis 75 Gew.-%, weiter bevorzugt von 6 bis 70 Gew.-%, noch weiter bevorzugt von 8 bis 60 Gew.-%, insbesondere von 8 bis 20 Gew.-%, bezogen auf das Gesamtgewicht des schweißhemmenden kosmetischen Mittels, enthalten.

Als Bestandteil a) der erfindungsgemäßen kosmetischen Mittel kann ebenfalls mindestens ein Riechstoff enthalten sein. Bevorzugt werden jedoch Mischungen verschiedener Riechstoffe verwendet, welche gemeinsam eine ansprechende Duftnote erzeugen. Im Rahmen der vorliegenden Erfindung einsetzbare Riechstoffe sind beispielsweise in der Offenlegungsschrift DE 10 2010 063 250 A1 offenbart.

Besonders ansprechend riechende erfindungsgemäße schweißhemmende kosmetische Mittel werden erhalten, wenn der mindestens eine Riechstoff in einer Gesamtmenge von 0,00001 bis 15 Gew.-%, vorzugsweise von 0,001 bis 9 Gew.-%, bevorzugt von 0,01 bis 8 Gew.-%, weiter bevorzugt von 0,1 bis 7 Gew.-%, noch weiter bevorzugt von 0,2 bis 6 Gew.-%, insbesondere von 0,2 bis 2 Gew.-%, bezogen auf das Gesamtgewicht des schweißhemmenden kosmetischen Mittels, enthalten ist.

Weiterhin können die erfindungsgemäßen schweißhemmenden kosmetischen Mittel als Bestandteil a) ein Wachs enthalten. Bevorzugt ist dieses Wachs ausgewählt aus der Gruppe von (i) Fettsäureglycerinmono-, -di- und -triestern; (ii) Butyrospermum Parkii (Shea Butter); (iii) Estern von gesättigten, einwertigen C₈₋₁₈-Alkoholen mit gesättigten C₁₂₋₁₈-Monocarbonsäuren; (iv) linearen, primären C_{12-C24}-Alkanolen; (v) Estern aus einem gesättigten, einwertigen C₁₆₋₆₀-Alkanol und einer gesättigten C_{8-C36}-Monocarbonsäure; (vi) Glycerintriestern von gesättigten linearen C₁₂₋₃₀-Carbonsäuren, die hydroxyliert sein können; (vii) natürlichen pflanzlichen Wachsen; (viii) tierischen Wachsen; (ix) synthetischen Wachsen; sowie (x) deren Mischungen. Im Rahmen der vorliegenden Erfindung bevorzugt einsetzbare Wachse sind in der Offenlegungsschrift DE 10 2012 222 692 A1 offenbart.

Im Rahmen der vorliegenden Erfindung ist es bevorzugt, wenn das Wachs in einer Gesamtmenge von 0,01 bis 50 Gew.-%, vorzugsweise von 3 bis 40 Gew.-%, bevorzugt von 5 bis 30 Gew-%, insbesondere von 6 bis 25 Gew.-%, bezogen auf das Gesamtgewicht des schweißhemmenden kosmetischen Mittels, enthalten ist.

Gemäß einer Ausführungsform der vorliegenden Erfindung kann es vorgesehen sein, dass die erfindungsgemäßen schweißhemmenden kosmetischen Mittel als Bestandteil b) ein Treibmittel in einer Gesamtmenge von 0 bis 99 Gew.-%, bezogen auf das Gesamtgewicht des schweißhemmenden kosmetischen Mittels, enthalten. Wenn die erfindungsgemäßen kosmetischen Mittel ein Treibmittel enthalten, ist dieses bevorzugt in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%, bezogen auf das Gesamtgewicht des schweißhemmenden kosmetischen Mittels, enthalten. In diesem Fall sind die erfindungsgemäßen kosmetischen Mittel als treibgasgetriebene Aerosole konfektioniert. Bevorzugte Treibmittel (Treibgase) sind Propan, Propen, n-Butan, iso-Butan, isoButen, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, Tetrafluoropropene und zwar sowohl einzeln als auch in deren Mischungen. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann.

Als dritten Bestandteil c) enthält das erfindungsgemäße schweißhemmende kosmetische Mittel mindestens ein spezielles Protein aus Malvengewächsen der Gattung *Adansonia* (wie hierin beschrieben). Die Gattung mit der lateinischen Bezeichnung *Adansonia* bezeichnet Laubbäume in Form von Affenbrotbäumen. Besonders bevorzugt werden erfindungsgemäß Proteine eingesetzt, welche aus dem afrikanischen Affenbrotbaum, auch als afrikanischer Baobab bezeichnet, isoliert werden können. Erfindungsgemässe Proteine sind solche welche aus den Samen dieser Bäume isoliert werden und hydrolysiert sind.

Eine besonders effektive Verminderung des Achselschweißes durch das mindestens eine spezielle Protein wird im Rahmen der vorliegenden Erfindung erreicht, wenn das mindestens eine Protein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%, bezogen auf das Gesamtgewicht des schweißhemmenden kosmetischen Mittels, enthalten ist. Ohne sich auf diese Theorie beschränken zu wollen resultiert der Einsatz der vorstehend genannten Mengen des mindestens einen speziellen Proteins in einer signifikanten Beeinflussung der Schweißdrüse(n) durch Geldbildung des Proteins in den Ausführungsgängen der Schweißdrüsen oder durch Beeinflussung des Ladungsgleichgewichts innerhalb der Schweißdrüse(n). Auf diese Weise wird eine hervorragende schweißhemmende Wirkung gewährleistet. Weiterhin führt der Einsatz der zuvor genannten Mengen des mindestens einen speziellen Proteins nicht zu instabilen Formulierungen, so dass die Stabilität der erfindungsgemäßen schweißhemmenden kosmetischen Mittel selbst über lange Lagerungszeiträume gewährleistet wird.

Besonders gute Ergebnisse im Hinblick auf die Verminderung und/oder Reduzierung der Achselnässe sowie auf die Hautverträglichkeit und die Lagerstabilität werden erhalten, wenn das mindestens eine Protein ein mittleres Molekulargewicht M_{w} von 150 bis 100.000 Da, vorzugsweise von 180 bis 50.000 Da, bevorzugt von 200 bis 10.000 Da, weiter bevorzugt von 250 bis 8.000 Da, insbesondere von 300 bis 5.000 Da, aufweist. Das mittlere Molekulargewicht M_{w} kann beispielsweise durch Gelpermeationschromatographie (GPC) bestimmt werden (Andrews P.; "Estimation of the Molecular Weights of Proteins by Sephadex Gel-Filtration"; Biochem. J., 1964, 91, Seiten 222 bis 233).

Gemäß einer bevorzugten Ausführungsform der vorliegenden Erfindung weist das mindestens eine Protein einen isoelektrischen Punkt auf, welcher im Bereich von pH 4,0 bis pH 10,0, vorzugsweise von pH 4,0 bis pH 9,5, insbesondere von pH 4,0 bis pH 8,0, liegt. Insbesondere Proteine, welche einen isoelektrischen Punkt im vorgenannten pH-Bereich aufweisen, haben sich im Rahmen der vorliegenden Erfindung als vorteilhaft im Hinblick auf die schweißhemmende Wirkung sowie die Stabilität der erfindungsgemäßen kosmetischen Mittel erwiesen.

Eine besonders hohe schweißhemmende Wirkung, Hautverträglichkeit sowie Lagerstabilität wird im Rahmen der vorliegenden Erfindung gewährleistet, wenn das mindestens eine Protein eine Veränderung der Lichtabsorption bei einer pH-Wert Änderung von mindestens 0,5 in einem pH-Bereich von pH 4,5 bis pH 7,5, insbesondere von pH 5,0 bis pH 7,0, bei einer Konzentration von 0,001 bis 10 Gew.-% Protein, bezogen auf das Gesamtgewicht der zur pH-Messung verwendeten Probenmischung, und einer Temperatur von 20 °C bewirkt. Ohne sich auf diese Theorie beschränken zu wollen resultiert der Einsatz des mindestens einen speziellen Proteins, welches in einem bestimmten pH-Bereich eine Veränderung der Lichtabsorption bewirkt, in einer signifikant erhöhten Beeinflussung der Schweißdrüse(n) durch pH-selektive Gelbildung in den Ausführungsgängen der Schweißdrüsen oder durch Störung des Ladungsgleichgewichts der Schweißdrüse(n). Auf diese Weise wird eine hervorragende schweißhemmende Wirkung der erfindungsgemäßen kosmetischen Mittel gewährleistet, welche mit der schweißhemmenden Wirkung von aluminiumsalzhaltigen oder aluminium-zirkoniumsalzhaltigen kosmetischen Mitteln des Standes der Technik vergleichbar ist.

Im Rahmen der vorliegenden Erfindung ist es bevorzugt, wenn die pH-Wert Änderung durch Zugabe von Hydrogencarbonaten oder Carbonaten, insbesondere von Natriumhydrogencarbonaten, erreicht wird.

Gemäß einer bevorzugten Ausführungsform der vorliegenden Erfindung ist das mindestens eine Protein ausgewählt aus der Gruppe von (i) nicht modifizierten Proteinen; (ii) hydrolysierten Proteinen; (iii) chemisch modifizierten Proteinen, insbesondere hydrophob und/oder kationisch und/oder anionisch modifizierten Proteinen; (iv) physikalisch modifizierten Proteinen, insbesondere fraktionierten und/oder gereinigten und/oder bestrahlten Proteinen; (v) hydrolysierten nicht modifizierten Proteinen; (vi) hydrolysierten und chemisch modifizierten Proteinen, insbesondere hydrolysierten und hydrophob und/oder kationisch und/oder anionisch modifizierten Proteinen; (vii) hydrolysierten und physikalisch modifizierten Proteinen, insbesondere fraktionierten und/oder gereinigten und/oder bestrahlten Proteinen; sowie (viii) deren Mischungen.

Unter dem Begriff "nicht modifizierte Proteine" sind erfindungsgemäß Proteine zu verstehen, welche weder mittels chemischer Verfahren, wie beispielsweise der Hydrolyse oder der chemischen Modifikation, noch mittels physikalischer Methoden, wie beispielsweise der Aufreinigung, der Trennung sowie der Bestrahlung, behandelt wurden.

Weiterhin sind unter dem Begriff "hydrolysierte Proteine" bzw. "Proteinhydrolysate" erfindungsgemäß Proteine zu verstehen, welche durch chemische, insbesondere alkalische oder saure, Hydrolyse, durch enzymatische Hydrolyse und/oder durch eine Kombination aus beiden Hydrolysearten hergestellt werden. Für den enzymatischen Abbau sind alle hydrolytisch wirkenden Enzyme geeignet, wie beispielsweise alkalische Proteasen. Übersichten zu Herstellung von Proteinhydrolysaten sind beispielsweise von G. Schuster und A. Domsch in Seifen Öle Fette Wachse 108, (1982) 177 bzw. Cosm.Toil. 99, (1984) 63, von H. W. Steisslinger in Parf.Kosm. 72, (1991) 556 und F. Aurich et al. in Tens.Surf.Det. 29, (1992) 389 erschienen. Mischungen von einzelnen Aminosäuren, welche lediglich durch Vermischen der Reinsubstanzen der Aminosäuren erhalten werden, sowie Totalhydrolysate, welche lediglich aus einzelnen Aminosäuren bestehen, fallen im Rahmen der vorliegenden Erfindung nicht unter den Begriff "hydrolysierte Proteine" bzw. "Proteinhydrolysate".

Darüber hinaus sind unter dem Begriff "chemisch modifizierte Proteine" im Rahmen der vorliegenden Erfindung Proteine zu verstehen, welche durch chemische Umsetzung der reaktiven Gruppen der Proteine, insbesondere der Hydroxy-, Amin-, Imidazol-, Guanidino- und/oder Thiolgruppen der Seitenketten der Aminosäuren des Proteins, mit hydrophoben und/oder kationischen und/oder anionischen Verbindungen erhalten werden.

Zudem sind unter dem Begriff "physikalisch modifizierte Proteine" im Sinne der vorliegenden Erfindung Proteine zu verstehen, welche durch physikalische Einwirkung, insbesondere durch Wärme und/oder Licht und/oder Fraktionierung, modifiziert wurden.

Im Rahmen dieser Ausführungsform ist es besonders bevorzugt, wenn das mindestens eine Protein ausgewählt ist aus der Gruppe von chemisch modifizierten, insbesondere hydrophob modifizierten, Proteinen. In diesem Zusammenhang weist das hydrophob modifizierte Protein ein oder mehrere C₄₋₃₀-Kohlenstoffketten auf, wobei die C₄₋₃₀-Kohlenwasserstoffketten linear, cyclisch, verzweigt, unverzweigt, gesättigt, ungesättigt und aromatisch sein können und wobei die C₄₋₃₀-Kohlenwasserstoffketten über Ether- und/oder Ester- und/oder Amin- und/oder Amidbindungen an den Proteinrest gebunden sind.

Darüber hinaus ist es im Rahmen dieser Ausführungsform bevorzugt, wenn das mindestens eine Protein ausgewählt ist aus der Gruppe von chemisch modifizierten, insbesondere kationisch modifizierten, Proteinen. Bevorzugt enthält das kationisch modifizierte Protein daher einen oder mehrere Rest(e) der Formel R¹-W(CH₃)₂-CH₂-CH(OH)-CH₂-X-R, in welcher R¹ für eine Alkylgruppe mit 1 bis 30 Kohlenstoffatomen, eine Alkenylgruppe mit 1 bis 30 Kohlenstoffatomen, eine Hydroxyalkylgruppe mit 1 bis 30 Kohlenstoffatomen, insbesondere für eine Methylgruppe, eine C₁₀₋₁₄-Alkyl oder eine C₁₀₋₁₄-Alkenylgruppe, X für O, N oder S und R für den Proteinrest stehen. Die Kationisierung der Proteine mit den vorstehend beschriebenen Resten kann durch Umsetzung der Proteine mit den entsprechenden Halogeniden der obigen Formel erreicht werden, wobei die vorstehend beschriebenen Reste über Ether- und/oder Ester- und/oder Amid- und/oder Aminbindungen an das Protein gebunden sein können. Unter dem Begriff "Proteinrest" ist im Rahmen der vorliegenden Erfindung das durch die Verknüpfung von Aminosäuren gebildete Rückgrat des entsprechenden Proteins, an welches die kationische Gruppe über die zuvor genannten Bindungen gebunden ist, zu verstehen.

Besonders gute Ergebnisse werden im Rahmen dieser Ausführungsform mit dem beschriebenen hydrolysierten Protein erhalten.

In diesem Zusammenhang hat es sich als vorteilhaft erwiesen, wenn das mindestens eine Protein einen Calciumgehalt von 300 bis 350 mg, einen Phosphorgehalt von 1.500 bis 1.700 mg, einen Schwefelgehalt von 410 bis 450 mg, jeweils bezogen auf 1 kg des Proteins, sowie einen Kaliumgehalt von 0,3 bis 0,5 Gew.-%, bezogen auf das Gesamtgewicht des Proteins, aufweist. Die zuvor angegebenen Gehalte können beispielsweise mittels Atomemissionsspektrometrie (ICP-OES) nach Mikrowellenaufschluss mit Salpetersäure ermittelt werden(A. Oliveira et. al; "Evaluation of Metal Ions in Rice Samples: Extraction and Direct Determination by ICP-OES"; J. Braz. Chem. Soc., 2012, 23, Seiten 838 bis 845).

Gemäß einer weiteren besonders bevorzugten Ausführungsform der vorliegenden Erfindung bewirkt das mindestens eine Protein eine Veränderung der Lichtabsorption von 1,5 bis 90 %, vorzugsweise von 2 bis 80 %, bevorzugt von 2,5 bis 70 %, weiter bevorzugt von 3 bis 65 %, insbesondere von 3,5 bis 60 %. Insbesondere Proteine aus Malvengewächsen der Gattung *Adansonia,* welche die zuvor genannte Veränderung der Lichtabsorption bewirken, führen im Rahmen der vorliegenden Erfindung zu einer hervorragenden schweißhemmenden Wirkung. Die Veränderung der Lichtabsorption kann dabei auf einer Veränderung der Lichtdurchlässigkeit der Probenmischung, insbesondere durch Trübung, als auch auf die Absorption von Licht durch die Probenmischung, insbesondere durch das Protein selbst, erfolgen.

Die dieser Erfindung zugrundeliegenden Veränderungen der Lichtabsorption bei einer pH-Wert Änderung von mindestens 0,5 können durch Messung der Lichttransmission eines Lichtstrahls durch die Probenmischung bestimmt werden. Die Messungen der Lichttransmission wird unter Verwendung einer Methrom Optrode 6.1115.000 bei einer Wellenlänge von 574 nm (grüngelb) in mV (Auflösung 0,1 mV) in einem offenen Probengefäß bei 23 °C und 1.013 mbar durchgeführt. Die pH-Wert Änderung in dem pH-Bereich von 4,0 bis 8,0 wird durch langsame und kontinuierliche Zugabe einer Carbonat- oder Hydrogencarbonatlösung, bevorzugt einer 1 Gew.-%-igen Natriumhydrogencarbonatlösung, zu der Probenmischung unter Messung des pH-Werts mit einer pH-Elektrode sowie unter Rühren bei einer Geschwindigkeit von 750 bis 850 rpm erreicht. Die Veränderung der Lichtabsorption, welche durch das mindestens eine Protein bewirkt wird, berechnet sich nach der Formel ΔL = [(|Li|/|L₀|]*100. In dieser Formel steht Lᵢ für die Lichttransmission vor und nach Veränderung des pH-Wertes um mindestens 0,5 in dem pH-Bereich von 4,0 bis 8,0, bevorzugt von pH 4,5 und 7,5, insbesondere von pH 5,0 und 7,0, also beispielsweise Lichttransmission bei pH 5,0 minus Lichttransmission bei pH 6,0. L₀ steht in dieser Formel für die Differenz aus der Lichttransmission bei pH 4,0 und bei pH 8,0, bevorzugt bei pH 4,5 und bei pH 7,5, insbesondere bei pH 5,0 und bei pH 7,0, also beispielsweise Lichttransmission bei pH 8,0 minus Lichttransmission bei pH 4,0. Das mindestens eine spezielle Protein in den erfindungsgemäßen schweißhemmenden kosmetischen Mitteln bewirkt eine nach obiger Methode bestimmte Veränderung der Lichtabsorption von 1 bis 100 %. Die vorliegende Erfindung ist jedoch nicht auf schweißhemmende kosmetische Zusammensetzungen beschränkt, welche mindestens ein spezielles Protein enthalten, welches eine nach obiger Methode bestimmte Veränderung der Lichtabsorption von 1 bis 100 % bewirkt. Sie umfasst auch schweißhemmende kosmetische Zusammensetzungen, welche mindestens ein spezielles Protein enthalten, welches nach anderen Methoden eine Veränderung der Lichtabsorption von 1 bis 100 % bewirkt.

Im Rahmen der vorliegenden Erfindung ist es bevorzugt, wenn die Konzentration des mindestens einen Proteins in der zur Bestimmung der Veränderung der Lichtabsorption verwendeten Mischung von 0,005 bis 10 Gew.-%, vorzugsweise von 0,05 bis 5 Gew.-%, bevorzugt von 0,07 bis 3 Gew.-%, insbesondere von 0,09 bis 2 Gew.-%, bezogen auf das Gesamtgewicht der für die Bestimmung der Veränderung der Lichtabsorption verwendeten Probenmischung, beträgt.

Erfindungsgemäß bevorzugt bewirkt das mindestens eine Protein eine Veränderung der Lichtabsorption bei einer pH-Wert Änderung von mindestens 0,5 und höchstens 3,5, vorzugsweise von mindestens 0,5 und höchstens 2,5, insbesondere von mindestens 0,5 und höchstens 1,5. Die Änderung des pH-Wertes kann insbesondere durch die Zugabe von Säuren oder Basen, bevorzugt Basen in Form von Carbonaten oder Hydrogencarbonaten, in der entsprechenden Menge erreicht werden.

Gemäß einer weiteren bevorzugten Ausführungsform der vorliegenden Erfindung weist das schweißhemmende kosmetische Mittel einen pH-Wert von pH 2 bis pH 10 auf. Innerhalb dieses Bereichs ist eine stabile Formulierung der erfindungsgemäßen kosmetischen Mittel möglich, ohne dass unerwünschte Wechselwirkungen zwischen den Inhaltsstoffen der erfindungsgemäßen schweißhemmenden kosmetischen Mittel auftreten. Die Einstellung des gewünschten pH-Wertes kann erfindungsgemäß durch Verwendung von dem Fachmann bekannten und in schweißhemmenden kosmetischen Mitteln üblichen Säuren und Basen erfolgen.

Erfindungsgemäß ist es weiterhin bevorzugt, wenn das schweißhemmende kosmetische Mittel zusätzlich mindestens ein Konservierungsmittel enthält. Erfindungsgemäß bevorzugte Konservierungsmittel sind Formaldehydabspalter lodopropinylbutylcarbamate, Parabene, Phenoxyethanol, Ethanol, Benzoesäure und deren Salze, Dibromdicyanobutan, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Salicylsäure und Salicylate. Weitere im Rahmen der vorliegenden Erfindung einsetzbare Konservierungsmittel sind die in Anlage 6 der Kosmetikverordnung aufgeführten Substanzen sowie kosmetische Rohstoffe mit konservierenden Eigenschaften oder Rohstoffe, welche die konservierende Wirkung der vorgenannten Konservierungsmittel unterstützen bzw. verstärken. Die Konservierungsmittel sind vorzugsweise in einer Gesamtmenge von 0,01 bis 10 Gew.-%, vorzugsweise von 0,1 bis 7 Gew.-%, bevorzugt von 0,2 bis 5 Gew.-%, insbesondere von 0,3 bis 2,0 Gew.-%, bezogen auf das Gesamtgewicht des schweißhemmenden kosmetischen Mittels, enthalten.

Im Rahmen der vorliegenden Erfindung ist es bevorzugt, wenn das schweißhemmende kosmetische Mittel als Wasser-in-ÖI-Emulsion vorliegt. Hierbei kann es sich insbesondere um eine versprühbare Wasser-in-ÖI-Emulsion handeln, welche mittels eines Treibmittels versprüht werden kann. In diesem Zusammenhang ist es bevorzugt, wenn das in Form einer Wasser-in-ÖI-Emulsion vorliegende erfindungsgemäße schweißhemmende kosmetische Mittel das mindestens eine Protein in einer Gesamtmenge von 0,1 bis 70 Gew.-%, vorzugsweise von 0,5 bis 60 Gew.-%, bevorzugt von 1,0 bis 50 Gew.-%, weiter bevorzugt von 1,5 bis 40 Gew-%, noch weiter bevorzugt von 2,0 bis 30 Gew.-%, insbesondere von 2,0 bis 20 Gew.-%, bezogen auf das Gesamtgewicht des schweißhemmenden kosmetischen Mittels, enthält.

Es kann jedoch erfindungsgemäß gleichermaßen bevorzugt sein, wenn das schweißhemmende kosmetische Mittel als ÖI-in-Wasser-Emulsion vorliegt. In diesem Fall wird das erfindungsgemäße kosmetische Mittel bevorzugt als treibmittelfreies Pumpspray oder Quetschspray versprüht oder als Roll-on appliziert. In diesem Zusammenhang ist es bevorzugt, wenn das in Form einer ÖI-in-Wasser-Emulsion vorliegende schweißhemmende kosmetische Mittel das mindestens eine Protein in einer Gesamtmenge von 0,1 bis 70 Gew.-%, vorzugsweise von 0,5 bis 60 Gew.-%, bevorzugt von 1,0 bis 50 Gew.-%, weiter bevorzugt von 1,5 bis 40 Gew-%, noch weiter bevorzugt von 2,0 bis 30 Gew.-%, insbesondere von 2,0 bis 20 Gew.-%, bezogen auf das Gesamtgewicht des schweißhemmenden kosmetischen Mittels, enthält.

Gemäß einer weiteren bevorzugten Ausführungsform der vorliegenden Erfindung können die erfindungsgemäßen kosmetischen Mittel nur einen geringen Gehalt an freiem Wasser bzw. kein freies Wasser enthalten. Unter freiem Wasser im Sinne der vorliegenden Erfindung wird Wasser verstanden, welches von Kristallwasser, Hydratationswasser oder ähnlich molekular gebundenem Wasser der eingesetzten Bestandteile verschieden ist. Das schweißhemmende kosmetische Mittel enthält bevorzugt freies Wasser in einer Gesamtmenge von weniger als 10 Gew.-%, vorzugweise von weniger als 8 Gew.-%, bevorzugt von weniger als 5 Gew.-%, weiter bevorzugt von weniger als 3 Gew.-%, noch mehr bevorzugt von weniger als 1 Gew.-%, insbesondere von 0 Gew.-%, bezogen auf das Gesamtgewicht des schweißhemmenden kosmetischen Mittels.

Im Rahmen einer weiteren Ausführungsform ist es jedoch erfindungsgemäß ebenfalls bevorzugt, wenn das schweißhemmende kosmetische Mittel als wässrige, wässrig-alkoholische oder wässrigglykolische Lösung vorliegt. Da die erfindungsgemäßen kosmetischen Mittel keine schweißhemmenden Halogenide und/oder Hydroxyhalogenide von Aluminium und/oder Zirconium enthalten, welche durch den Zusatz protischer Lösungsmittel eine verminderte schweißhemmende Wirkung aufweisen, können erfindungsgemäß protische Lösungsmittel, wie wässrige Lösungen, zur Formulierung der erfindungsgemäßen schweißhemmenden kosmetischen Mittel verwendet werden, ohne dass eine signifikante Verringerung der Antitranspirantwirkung auftritt. Daher gewährleistet der Zusatz des mindestens einen speziellen Proteins selbst bei Verwendung von protischen Lösungsmitteln eine effektive Beeinflussung der der Schweißdrüse(n) und somit eine hervorragende Antitranspirantwirkung.

Im Zusammenhang mit dieser Ausführungsform der vorliegenden Erfindung wurde überraschenderweise festgestellt, dass die Beeinflussung der Schweißdrüse(n) durch das mindestens eine spezielle Protein signifikant gesteigert werden kann, wenn die erfindungsgemäßen schweißhemmenden kosmetischen Mittel freies Wasser in einer Menge von 5 bis 99 Gew.-%, bezogen auf das Gesamtgewicht des schweißhemmenden kosmetischen Mittels, enthalten. In einer besonders bevorzugten Ausführungsform der vorliegenden Erfindung enthält das schweißhemmende kosmetische Mittel daher freies Wasser in einer Gesamtmenge von 5 bis 96 Gew.-%, vorzugsweise von 15 bis 80 Gew.-%, bevorzugt von 30 bis 70 Gew.-%, insbesondere von 40 bis 60 Gew.-%, bezogen auf das Gesamtgewicht des schweißhemmenden kosmetischen Mittels.

Weiterhin ist es im Zusammenhang mit dieser Ausführungsform bevorzugt, wenn das schweißhemmende kosmetische Mittel Ethanol in einer Gesamtmenge von 1 bis 99 Gew.-%, vorzugsweise von 5 bis 70 Gew.-%, bevorzugt von 7 bis 50 Gew.-%, insbesondere von 10 bis 30 Gew.-%, bezogen auf das Gesamtgewicht des schweißhemmenden kosmetischen Mittels, enthält. Wie zuvor ausgeführt, können durch den Einsatz des mindestens einen speziellen Proteins selbst hohe Mengen an protischen Lösungsmitteln, wie Ethanol, verwendet werden, ohne dass die Antitranspirantwirkung des erfindungsgemäßen schweißhemmenden kosmetischen Mittels negativ beeinflusst wird.

Die Applikation des erfindungsgemäßen schweißhemmenden kosmetischen Mittels kann mittels verschiedener Verfahren erfolgen. Gemäß einer bevorzugten Ausführungsform ist das schweißhemmende kosmetische Mittel als Spray-Applikation konfektioniert. Die Spray-Applikation erfolgt mit einer Sprühvorrichtung, welche in einem Behälter eine Füllung aus dem erfindungsgemäßen flüssigen, viskos-fließfähigen, suspensionsförmigen oder pulverförmigen schweißhemmenden kosmetischen Mittel enthält. Die Füllung kann unter dem Druck eines Treibmittels stehen (Druckgasdosen, Druckgaspackungen, Aerosolpackungen), oder es kann sich um einen mechanisch zu bedienenden Pumpzerstäuber ohne Treibgas (Pumpsprays/ Quetschflasche) handeln. Die Zerstäubung des schweißhemmenden kosmetischen Mittels kann hierbei physikalisch, mechanisch oder elektromechanisch, beispielsweise durch Piezzoeffekte oder elektrische Pumpen, erfolgen. Im Rahmen dieser Ausführungsform einsetzbare Behälter und Entnahmevorrichtungen sind beispielsweise in der Offenlegungsschrift DE 10 2012 222 692 A1 beschrieben.

Das schweißhemmende kosmetische Mittel kann weiterhin bevorzugt als Stift, Soft Solid, Creme, Gel, Roll-on, loses oder kompaktes Puder konfektioniert sein. Die Formulierung der erfindungsgemäßen schweißhemmenden kosmetischen Mittel in einer bestimmten Darreichungsform, wie beispielsweise einem Antitranspirant-Roll-on, einem Antitranspirantstift oder einem Antitranspirantgel, richtet sich bevorzugt nach den Anforderungen des Verwendungszwecks. Je nach Verwendungszweck können die erfindungsgemäßen schweißhemmenden kosmetischen Mittel daher in fester, halbfester, flüssiger, disperser, emulgierter, suspendierter, gelförmiger, mehrphasiger oder puderförmiger Form vorliegen. Unter den Begriff der Flüssigkeit fallen im Sinne der vorliegenden Erfindung auch jegliche Arten von Festkörperdispersionen in Flüssigkeiten. Weiterhin werden unter mehrphasigen erfindungsgemäßen schweißhemmenden kosmetischen Mitteln im Sinne der vorliegenden Erfindung Mittel verstanden, welche mindestens 2 verschiedene Phasen mit einer Phasentrennung aufweisen und bei welchen die Phasen horizontal, also übereinander, oder vertikal, also nebeneinander, angeordnet sein können. Die Applikation kann beispielsweise mit einem Rollkugelapplikator oder mittels eines festen Stifts erfolgen.

Es kann im Rahmen der vorliegenden Erfindung ebenfalls bevorzugt sein, wenn das schweißhemmende kosmetische Mittel auf und/oder in einem wegwerfbaren Substrat, ausgewählt aus der Gruppe von Tüchern, Pads und Bauschen, enthalten ist. Besonders bevorzugt sind Feuchttücher, d.h. für den Anwender vorgefertigte, bevorzugt einzeln abgepackte, Feuchttücher, wie sie z. B. aus dem Bereich der Glasreinigung oder aus dem Bereich der feuchten Toilettenpapiere wohlbekannt sind. Solche Feuchttücher, die vorteilhafter Weise auch Konservierungsstoffe enthalten können, sind mit einem erfindungsgemäßen schweißhemmenden kosmetischen Mittel imprägniert oder beaufschlagt und bevorzugt einzeln verpackt. Bevorzugte Substratmaterialien sind ausgewählt aus porösen flächigen Tüchern. Zu diesen Tüchern gehören Tücher aus gewebten und ungewebten (Vlies) synthetischen und natürlichen Fasern, Filz, Papier oder Schaumstoff, wie hydrophilem Polyurethanschaum. Erfindungsgemäß bevorzugte deodorierende oder schweißhemmende Substrate können durch Tränken oder Imprägnierung oder auch durch Aufschmelzen eines erfindungsgemäßen schweißhemmenden kosmetischen Mittels auf ein Substrat erhalten werden.

Erfindungsgemäß bevorzugt enthält das schweißhemmende kosmetische Mittel mindestens einen weiten Hilfsstoff, ausgewählt aus der Gruppe von (i) Emulgatoren und/oder Tensiden; (ii) Verdickungsmitteln; (iii) Chelatbildnern; (iv) Deodorant-Wirkstoffen; (v) ein- und/oder mehrwertigen Alkoholen und/oder Polyethylenglycolen; (vi) hautkühlenden Wirkstoffen; (vii) pH-Stellmitteln; (viii) hautpflegenden Wirkstoffen, wie Moisturizern, hautberuhigenden Stoffen, hautaufhellenden Stoffen, hautglättenden Stoffen; sowie (ix) deren Mischungen.

Erfindungsgemäß bevorzugt geeignete Emulgatoren und Tenside sind ausgewählt aus anionischen, kationischen, nichtionischen, amphoteren, insbesondere ampholytischen und zwitterionischen Emulgatoren und Tensiden. Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8 bis 28 Kohlenstoffatomen, die gesättigt oder ungesättigt, linear oder verzweigt sein kann. Besonders bevorzugt ist diese C₈-C₂₈-Alkylkette linear. Im Rahmen der vorliegenden Erfindung bevorzugt einsetzbare Emulgatoren und Tenside sind beispielsweise in den Offenlegungsschriften DE 10 2012 222 692 A1, DE 10 2010 063 250 A1 sowie DE 10 2010 055 816 A1 offenbart.

Zur Verdickung der erfindungsgemäßen schweißhemmenden kosmetischen Mittel werden bevorzugt Substanzen eingesetzt, welche ausgewählt sind aus Celluloseethern, Xanthan-Gum, Sclerotium Gum, Succinoglucanen, Polygalactomannanen, Pectinen, Agar, Carragheen (Carrageenan), Traganth, Gummi arabicum, Karayagummi, Taragummi, Gellan, Gelatine, Propylenglycolalginat, Alginsäuren und deren Salze, Polyvinylpyrrolidonen, Polyvinylalkoholen, Polyacrylamiden, physikalisch (z. B. durch Vorverkleisterung) und/oder chemisch modifizierten Stärken, Acrylsäure-Acrylat-Copolymeren, Acrylsäure-Acrylamid-Copolymeren, Acrylsäure-Vinylpyrrolidon-Copolymeren, Acrylsäure-Vinylformamid-Copolymeren und Polyacrylaten. Besonders bevorzugte Verdickungsmittel sind weiterhin ausgewählt aus Carbomeren. Carbomere sind verdickende vernetzte Polymere aus Acrylsäure, Methacrylsäure sowie deren Salzen. Die Vernetzung kann mittels polyfunktioneller Verbindungen wie Polyalkylenether von Polysacchariden oder Polyalkoholen, beispielsweise Sucroseallylether, Pentaerythritolallylether, Propylenallylether, erfolgen. Bevorzugt im Rahmen der vorliegenden Erfindung sind Homopolymere von Acrylsäure oder deren Salzen, welche mit einem Pentaerythritolallylether, einem Sucroseallylether oder einem Propylenallylether vernetzt sind. Ein im Rahmen der vorliegenden Erfindung einsetzbares Verdickungsmittel ist ein Copolymer aus C₁₀₋₃₀-Alkylacrylat, Acrylsäure, Methacrylsäure sowie deren Estern, welches mit einem Sucroseallylether oder einem Pentaerythritolallylether vernetzt ist. Verdickungsmittel auf Basis von Carbomeren sind die unter dem Handelsnamen Carbopol® (BF Goodrich, Ohio, USA) erhältlichen Produkte wie beispielsweise Carbopol 934, Carbopol 940, Carbopol 941, Carbopol 971, Carbopol 974, Carbopol EZ2, Carbopol ETD 2001, Carbopol ETD 2020, Carbopol ETD 2050, Carbopol ultrez 10, Carbopol ultrez 20, oder Carbopol ultrez 21.

Weiterhin können zur Verdickung der erfindungsgemäßen schweißhemmenden kosmetischen Mittel lipophile Verdickungsmittel eingesetzt werden. Erfindungsgemäß bevorzugte lipophile Verdickungsmittel sind ausgewählt aus hydrophobierten Tonmineralien, Bentoniten, pyrogenen Kieselsäuren sowie deren Derivaten.

Um die Beeinflussung der Schweißdrüse(n) durch das mindestens eine spezielle Protein weiter zu unterstützen, kann es von Vorteil sein, den erfindungsgemäßen schweißhemmenden kosmetischen Mitteln mindestens einen Chelatbildner, in einer Gesamtmenge von 0,01 bis 3,0 Gew.-%, vorzugsweise von 0,02 bis 1,0 Gew.-%, insbesondere von 0,05 bis 0,1 Gew.-%, bezogen auf das Gesamtgewicht des erfindungsgemäßen schweißhemmenden Mittels, zuzusetzen. Im Rahmen der vorliegenden Erfindung sind bevorzugte Chelatbildner ausgewählt aus der Gruppe von β-Alanindiessigsäure, Cyclodextrin, Diethylentriaminpentamethylenphosphonsäure, Natrium-, Kalium-, Calciumdinatrium-, Ammonium- und Triethanolaminsalzen der Ethylendiamintetraessigsäure (EDTA), Etidronsäure, Hydroxyethylethylendiamintetraessigsäure (HEDTA) und ihren Natriumsalzen, Natriumsalzen der Nitrilotriessigsäure (NTA), Diethylentriaminpentaessigsäure, Phytinsäure, Hydroxypropylcyclodextrin, Methylcyclodextrin, Aminotrimethylenphosphonat-Pentanatrium, Ethylendiamintetramethylenphosphonat-Pentanatrium, Diethylentriamin-pentaacetat-Pentanatrium, Pentanatriumtriphosphat, Kalium-EDTMP, Natrium-EDTMP, Natriumdihydroxyethylglycinat, Natriumphytat, Natriumpolydimethylglycinophenolsulfonat, Tetrahydroxyethylethylendiamin, Tetrahydroxypropylethylendiamin, Tetrakaliumetidronat, Tetranatriumetidronat, Tetranatriumiminodisuccinat, Trinatriumethylendiamindisuccinat, Tetranatrium-N,N-bis(Carboxymethyl)glutamat, Tetranatrium-DL-Alanin-N,N-diacetat und Desferrioxamin.

Die desodorierende Wirkung der erfindungsgemäßen schweißhemmenden kosmetischen Mittel kann weitergehend gesteigert werden, wenn mindestens ein Deodorant-Wirkstoff mit antibakterieller und/oder bakteriostatischer und/oder enzyminhibierender und/oder geruchsneutralisierender und/oder geruchsabsorbierender Wirkung in einer Gesamtmenge von 0,0001 bis 40 Gew.-%, vorzugsweise von 0,2 bis 20 Gew.-%, bevorzugt von 1 bis 15 Gew.-%, insbesondere von 1,5 bis 5 Gew.-%, bezogen auf das Gesamtgewicht des erfindungsgemäßen schweißhemmenden kosmetischen Mittels, enthalten ist. Sofern Ethanol in den erfindungsgemäßen Mitteln eingesetzt wird, gilt dieses im Rahmen der vorliegenden Erfindung nicht als Deodorant-Wirkstoff, sondern als Bestandteil des Trägers. Erfindungsgemäß bevorzugte Deodorant-Wirkstoffe sind beispielsweise in der Offenlegungsschrift DE 10 2010 063 250 A1 offenbart.

Bevorzugte erfindungsgemäße schweißhemmenden kosmetische Mittel enthalten weiterhin mindestens ein wasserlösliches mehrwertiges C₂₋₉-Alkanol mit 2 bis 6 Hydroxylgruppen und/oder mindestens ein wasserlösliches Polyethylenglycol mit 3 bis 50 Ethylenoxid-Einheiten sowie Mischungen hiervon. Hierunter fallen nicht die vorstehend erwähnten Deodorant-Wirkstoffe in Form von 1,2-Alkandiolen. Bevorzugte Alkanole und wasserlösliche Polyethylenglycole sind beispielsweise in der Offenlegungsschrift DE 10 2010 063 250 A1 beschrieben.

Gemäß einer weiteren Ausführungsform der vorliegenden Erfindung enthalten die schweißhemmenden kosmetischen Mittel weiterhin mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthyl Ethyl Oxamate, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat, 2-Hydroxymethyl-3,5,5-trimethylcyclohexanol und 5-Methyl-2-(1-methylethyl)cyclohexyl-N-ethyloxamat. Als hautkühlende Wirkstoffe bevorzugt sind Menthol, Isopulegol, Menthyllactat, Menthoxypropandiol, Menthylpyrrolidoncarbonsäure und 5-Methyl-2-(1-methylethyl)cyclohexyl-N-ethyloxamat sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Als pH-Stellmittel kommen erfindungsgemäß bevorzugt Säuren und/oder Alkalisierungsmittel und/oder Puffer zum Einsatz. Als Säuren werden erfindungsgemäß bevorzugt anorganische Säuren (wie beispielsweise Salzsäure, Schwefelsäure oder Phosphorsäure) oder organische Säuren (wie beispielsweise Zitronensäure, Weinsäure oder Apfelsäure) eingesetzt. Die erfindungsgemäß verwendbaren Alkalisierungsmittel werden bevorzugt ausgewählt aus der Gruppe, welche gebildet wird, aus Ammoniak, basischen Aminosäuren, Alkalihydroxiden, Carbonaten und Hydrogencarbonaten, Alkanolaminen, beispielsweise Amino-2-methyl-1-propanol, Monoethanolamin, Triethanolamin, Diethanolamin und Triisopropanolamin, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium. Als Puffersysteme eignen sich im Rahmen der vorliegenden Erfindung insbesondere Kohlensäure-Bicarbonat-Puffer, Kohlensäure-Silicat-Puffer, Essigsäure-Acetat-Puffer, Phosphatpuffer, Ammoniakpuffer, Citronensäure- oder Citratpuffer, Puffer auf Basis von Tris(hydroxymethyl)-aminomethan, Puffer auf Basis von 4-(2-Hydroxyethyl)-1-piperazinethanesulfonsäure, Puffer auf Basis von 4-(2-Hydroxyethyl)-piperazin-1-propansulfonsäure, Puffer auf Basis von 2-(N-Morpholino)ethansulfonsäure sowie Barbital-Acetat-Puffer. Die Wahl des entsprechenden Puffersystems richtet sich hierbei nach dem gewünschten pH-Wert der erfindungsgemäßen schweißhemmenden kosmetischen Mittel.

Die nachfolgend beschriebenen bevorzugten Ausführungsformen enthalten keine schweißhemmenden Halogenide und/oder Hydroxyhalogenide von Aluminium und/oder Zirconium:
In einer bevorzugten Ausführungsform sind die erfindungsgemäßen schweißhemmenden kosmetischen Mittel dadurch gekennzeichnet, dass sie - bezogen auf das Gesamtgewicht des erfindungsgemäßen schweißhemmenden kosmetischen Mittels -
- mindestens ein Protein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens einen Emulgator und/oder ein Tensid,
- mindestens ein pH-Stellmittel,
- mindestens ein Konservierungsmittel und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Protein in Malvengewächsen der Gattung *Adansonia* vorkommt und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

In einer weiteren bevorzugten Ausführungsform sind die erfindungsgemäßen schweißhemmenden kosmetischen Mittel dadurch gekennzeichnet, dass sie - bezogen auf das Gesamtgewicht des erfindungsgemäßen schweißhemmenden kosmetischen Mittels -
- mindestens ein Protein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens einen Emulgator und/oder ein Tensid,
- mindestens ein pH-Stellmittel,
- mindestens ein Konservierungsmittel,
- 0,01 bis 2 Gew.-%, vorzugsweise 0,1 bis 1 Gew.-%, bevorzugt 0,2 bis 0,7 Gew.-%, insbesondere 0,3 bis 0,5 Gew.-% eines Verdickungsmittels, und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Protein in Malvengewächsen der Gattung *Adansonia* vorkommt und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

In einer bevorzugten Ausführungsform sind die erfindungsgemäßen schweißhemmenden kosmetischen Mittel dadurch gekennzeichnet, dass sie - bezogen auf das Gesamtgewicht des erfindungsgemäßen schweißhemmenden kosmetischen Mittels -
- mindestens ein Protein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens ein Treibmittel in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%,
- mindestens einen Emulgator und/oder ein Tensid,
- mindestens ein pH-Stellmittel,
- mindestens ein Konservierungsmittel und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Protein in Malvengewächsen der Gattung *Adansonia* vorkommt und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

In einer weiteren bevorzugten Ausführungsform sind die erfindungsgemäßen schweißhemmenden kosmetischen Mittel dadurch gekennzeichnet, dass sie - bezogen auf das Gesamtgewicht des erfindungsgemäßen schweißhemmenden kosmetischen Mittels -
- mindestens ein Protein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens ein Treibmittel in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%,
- mindestens einen Emulgator und/oder ein Tensid,
- mindestens ein pH-Stellmittel,
- mindestens ein Konservierungsmittel,
- 0,01 bis 2 Gew.-%, vorzugsweise 0,1 bis 1 Gew.-%, bevorzugt 0,2 bis 0,7 Gew.-%, insbesondere 0,3 bis 0,5 Gew.-% eines Verdickungsmittels, und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Protein in Malvengewächsen der Gattung *Adansonia* vorkommt und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

Im Rahmen der vorliegenden Erfindung kann es auch vorgesehen sein, das erfindungsgemäße kosmetische Mittel im Rahmen eines Zwei-Komponenten-Mittels einzusetzen. Die einzelnen Komponenten werden hierzu vorzugsweise in getrennten Containern gelagert und in beliebiger Reihenfolge nacheinander oder gleichzeitig auf die Haut aufgetragen.

Ein weiterer Gegenstand der vorliegenden Erfindung ist daher eine Verpackungseinheit (Kit-of-Parts), umfassend - getrennt voneinander konfektioniert -
a) mindestens einen ersten Container (C1), enthaltend ein kosmetisches Mittel (M1) umfassend mindestens einen schweißhemmenden Wirkstoff, und
b) mindestens einen zweiten Container (C2), enthaltend ein kosmetisches Mittel (M2) gemäß der Ansprüche und wobei das kosmetische Mittel keine Halogenide und/oder Hydroxyhalogenide von Aluminium und/oder Zirconium enthält.

Unter dem Begriff "schweißhemmender Wirkstoff werden erfindungsgemäß Wirkstoffe verstanden, welche die Transpiration der Schweißdrüsen des Körpers vermindern bzw. reduzieren. Hierunter fallen jedoch nicht die in dem kosmetischen Mittel (M2) enthaltenen Proteine aus Malvengewächsen der Gattung *Adansonia,* welche unter den oben beschriebenen Bedingungen eine Veränderung der Lichtabsorption bewirken.

Bezüglich des kosmetischen Mittels (M2) in dem Container (C2) gilt mutatis mutandis das zu den erfindungsgemäßen kosmetischen Mitteln Gesagte.

Ein weiterer Gegenstand der vorliegenden Erfindung ist die Verwendung eines Proteins zur zumindest teilweisen Beeinflussung der Schweißdrüse(n), wobei das mindestens eine Protein in Malvengewächsen der Gattung *Adansonia* vorkommt und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

Unter Beeinflussung der Schweißdrüse(n) ist erfindungsgemäß die Beeinflussung der Schweißdrüse(n) dahingehend zu verstehen, dass die Absonderung von Schweiß aus dem Ausführungsgang vermieden bzw. verringert wird. Ohne sich auf eine Theorie beschränken zu wollen kann dies beispielsweise durch Bildung eines Gels und/oder Niederschlags des mindestens einen speziellen Proteins in dem Ausführungsgang der Schweißdrüse bzw. den Ausführungsgängen der Schweißdrüsen geschehen. Weiterhin kann der Einsatz des mindestens einen speziellen Proteins jedoch auch zu einer Störung des Ladungsgleichgewichts innerhalb der Ausführungsgänge der Schweißdrüsen führen. Bezüglich der erfindungsgemäßen Verwendung gilt mutatis mutandis das zu den erfindungsgemäßen kosmetischen schweißhemmenden Mitteln Gesagte.

Zudem ist ein weiterer Gegenstand der vorliegenden Erfindung ein schweißhemmendes kosmetisches Mittel, enthaltend
a) mindestens einen Stoff, ausgewählt aus der Gruppe von kosmetischen Ölen, welche bei 20 °C und 1.013 hPa flüssig sind, Riechstoffen und Wachsen,
b) Treibmittel in einer Gesamtmenge von 0 bis 99 Gew.-%, bezogen auf das Gesamtgewicht des schweißhemmenden kosmetischen Mittels, und
c) mindestens ein aus Malvengewächsen der Gattung *Adansonia* isoliertes Protein, bevorzugt ein aus den Samen der Gattung *Adansonia* isoliertes Protein, in einer Gesamtmenge von 2 bis 40 Gew.-%, bezogen auf das Gesamtgewicht des schweißhemmenden kosmetischen Mittels, wobei das Protein hydrolysiert ist,
wobei das schweißhemmende kosmetische Mittel keine Halogenide und/oder Hydroxyhalogenide von Aluminium und/oder Zirconium enthält.

Im Rahmen dieses Gegenstands ist es besonders bevorzugt, wenn das mindestens eine Protein einen Calciumgehalt von 300 bis 350 mg, einen Phosphorgehalt von 1.500 bis 1.700 mg, einen Schwefelgehalt von 410 bis 450 mg, jeweils bezogen auf 1 kg des Proteins, sowie einen Kaliumgehalt von 0,3 bis 0,5 Gew.-%, bezogen auf das Gesamtgewicht des Proteins, aufweist. Die zuvor angegebenen Gehalte können beispielsweise mittels Atomemissionsspektrometrie (ICP-OES), wie zuvor angeführt, ermittelt werden.

Bezüglich dieses Gegenstands der vorliegenden Erfindung gilt mutatis mutandis das zu den erfindungsgemäßen schweißhemmenden kosmetischen Mitteln sowie zu der erfindungsgemäßen Verwendung Gesagte.

Schließlich ist ein weiterer Gegenstand der vorliegenden Erfindung ein nicht-therapeutisches kosmetisches Verfahren zur Verhinderung und/oder Reduzierung der Transpiration des Körpers, bei welchem ein erfindungsgemäßes schweißhemmendes kosmetisches Mittel oder eine erfindungsgemäße Verpackungseinheit auf die Haut, insbesondere auf die Haut der Achselhöhlen, aufgetragen wird und für mindestens 1 Stunde, vorzugsweise für mindestens 2 Stunden, bevorzugt für mindestens 4 Stunden, insbesondere für mindestens 6 Stunden, auf der Haut der Achselhöhlen verbleibt.

Falls im Rahmen des erfindungsgemäßen Verfahrens die Verpackungseinheit gemäß der Erfindung eingesetzt wird, kann es vorgesehen sein, dass zunächst das kosmetische Mittel (M1) in dem Container (C1) und anschließend das kosmetische Mittel (M2) in dem Container (C2) aufgetragen wird. Es ist jedoch auch möglich, dass zunächst das kosmetische Mittel (M2) in dem Container (C2) und anschließend das kosmetische Mittel (M1) in dem Container (C1) aufgetragen wird. Darüber hinaus können das kosmetische Mittel (M1) in dem Container (C1) und das kosmetische Mittel (M2) in dem Container (C2) auch gleichzeitig aufgetragen werden. Die Zeitspanne zwischen der Anwendung der beiden Mittel (M1) und (M2) beträgt von 0 Sekunden bis 24 Stunden.

Bezüglich dieses Gegenstands der vorliegenden Erfindung gilt mutatis mutandis das zu den erfindungsgemäßen schweißhemmenden kosmetischen Mitteln, der erfindungsgemäßen Verpackungseinheit sowie zu der erfindungsgemäßen Verwendung Gesagte.

Die folgenden Beispiele erläutern die vorliegende Erfindung:

### Beispiele:

### 1. Veränderung der Lichtabsorption

Es wurde ein aus den Samen der Gattung *Adansonia* isoliertes hydrolysiertes Protein verwendet. Die Bestimmung der Veränderung der Lichtabsorption, welche durch die zuvor genannten Proteine in einem pH-Bereich von 4,0 bis 8,0 bei einer pH-Wert Änderung von mindestens 0,5 bewirkt wird, wird folgendermaßen durchgeführt:

**Tabelle 1: Probenlösung (Angaben in Gew.-%)**

| | E-I* |
|---|---|
| Hydrolysiertes Protein der Gattung *Adansonia* ^{a)} | 10 |
| HCl | Add pH |
| Wasser | Add 100 |

| | |
|---|---|
| * erfindungsgemäß a) Baobab Tein NPNF; (INCl: Hydrolyzed Adnasonia Digitata Seed Protein; 10 Gew.-%ige Lösung in Wasser; Neochem) | |

Für die Bestimmung der Veränderung der Lichtabsorption wurde ein Methrom Titrando 905 der Firma Methrom (USA) verwendet, welches mit einer Methrom Optrode 6.1115.000 sowie einer pH-Elektrode von Methrom ausgestattet ist. Die Steuerung des Methrom Titrando 905 erfolgt über die Software Tiamo der Firma Methrom. Zunächst wurden 30 ml einer Probenlösung gemäß Tabelle 1, welche einen pH-Wert von 3,0 aufwies, in dem offenen Probengefäß des Methrom Titrando 905 vorgelegt. Anschließend wurde bei 23 °C und 1.013 mbar unter Rühren (Rührgeschwindigkeit 8 des Titrando 905, entspricht ca. 750 bis 850 rpm) solange kontinuierlich eine 1 Gew.-%-ige Natriumhydrogencarbonatlösung zugegeben, bis ein pH-Wert von 7,5 erreicht wurde. Während der Zugabe der 1 Gew.-%-igen Natriumhydrogencarbonatlösung wurde die Lichttransmission eines Lichtstrahls durch diese Probenlösung mit einer Methrom Optrode 6.1115.000 bei einer Wellenlänge von 574 nm (grüngelb) in mV (Auflösung 0,1 mV) gemessen. Jede Messung wird jeweils zweimal durchgeführt und hieraus der Mittelwert gebildet.

Die Veränderung der Lichtabsorption, welche durch die oben genannten Peptide bewirkt wurde, wurde gemäß der Formel ΔL = [(|Lᵢ|/L₀|]*100 bestimmt. In dieser Formel steht Lᵢ für die Differenz aus der Lichttransmission vor und nach Veränderung des pH-Wertes um mindestens 0,5 in dem pH-Bereich von 4,0 bis 8,0, bevorzugt von pH 4,5 und 7,5, insbesondere von pH 5,0 und 7,0. Lo steht in dieser Formel für die Differenz aus der Lichttransmission bei pH 4,0 und bei pH 8,0, bevorzugt bei pH 4,5 und bei pH 7,5, insbesondere bei pH 5,0 und bei pH 7,0.

Bei einer pH-Wert Änderung von 1,0 zwischen pH 5,0 und pH 6,0 (Differenz Lichtabsorption bei pH 5,5 minus pH 6,0 bildet Wert Lᵢ) in einem pH-Wertbereich von 4,5 bis 7,5 (Differenz Lichtabsorption bei pH 7,5 minus pH 4,5 bildet Wert L₀) bewirkte dieses Protein eine Veränderung der Lichtabsorption ΔL von 40 %.

### 2. In-vivo Test zur Antitranspirantwirkung

Zur Ermittlung der Antitranspirantwirkung wurde eine Antitranspirantstudie auf dem Rücken von 16 Probandinnen durchgeführt. Hierzu wurden die folgenden schweißhemmenden Mittel verwendet:

| Schweißhemmendes Mittel | Nr |
|---|---|
| Wässrige Lösung mit 10 % ACH, pH 4 | V-I |
| Wässrige Lösung mit 2 %* Protein ^{a)}, pH 2-4 | E-II** |

| | |
|---|---|
| *Aktivsubstanz ** erfindungsgemäß ^{a)} Baobab Tein NPNF; (INCl: Hydrolyzed Adnasonia Digitata Seed Protein; 10 Gew.-%ige Lösung in Wasser; Neochem) | |

Auf dem Rücken von 16 Probandinnen wurden jeweils auf einer Seite neben dem Rückgrat 40 µL des schweißhemmenden Mittels V-I und 75 µL des erfindungsgemäßen kosmetischen Mittels E-II aufgetragen. Nach 5 Minuten wurden die behandelten Stellen mit okklusiver nichtadsorbierender Folie abgedeckt. Nach 2 Stunden wurden diese nichtadorbierenden Pads entfernt. Die Zusammensetzungen wurden an vier aufeinanderfolgenden Tagen jeweils auf die zuvor beschriebene Art und Weise auf den Rücken der Probandinnen aufgetragen. 24 h nach dem letzten Auftragen der Zusammensetzung wurden auf dem Rücken der Probandinnen saugfähige Pads an den Stellen aufgebracht, wo zuvor die Zusammensetzungen appliziert wurden. Weiterhin wurden auf der anderen Seite des Rückgrats in der gleichen Höhe ebenfalls Pads aufgebracht, welche als Kontrolle dienten. Nachdem die Probandinnen für ca. 15 Minuten bei 80 °C in der Sauna geschwitzt hatten, wurde die Menge des durch die Pads aufgenommenen Schweißes gravimetrisch bestimmt, wobei jede Zusammensetzung mit der jeweils korrespondierenden unbehandelten Stelle auf dem Rücken verglichen wurde. Aus der gravimetrischen Bestimmung der Schweißmenge wurde die Schweißreduktion ermittelt, wobei alle ermittelten Werte statistisch signifikant waren.

Die Schweißreduktion der jeweiligen Zusammensetzung im Vergleich zu einer unbehandelten Hautstelle ist in der nachfolgenden Tabelle wiedergegeben:

| Nr | Schweißreduktion |
|---|---|
| V-I | 50 % |
| E-II | 25% |

Die Verwendung des speziellen Proteins führt zu einer signifikanten Verminderung der Schweißreduktion und zu einer zufriedenstellenden Antitranspirantwirkung.

### 3. Formulierungen

Das in den nachfolgenden Beispielen eingesetzte Protein ist bevorzugt ein aus den Samen der Gattung *Adansonia* isoliertes hydrolysiertes Protein.

Erfindungsgemäße schweißhemmende kosmetische Mittel mit einem pH von 2,5 bis 10,0 (Mengenangaben in Gew.-%)

| | 1 | 2 | 3 | 4 | 5 | 6 | 7 |
|---|---|---|---|---|---|---|---|
| Isopropylmyristat | 0,50 | 0,10 | 0,50 | 1,0 | 2,0 | 3,0 | 5,0 |
| Protein | 0,50 | 2,0 | 3,0 | 5,0 | 7,0 | 10 | 20 |
| Eumulgin B3 ^{b)} | 3,0 | 3,0 | 3,0 | 4,0 | 4,0 | 4,0 | 5,0 |
| Parfum | 0,10 | 0,20 | 0,30 | 0,30 | 0,50 | 0,8 | 1,0 |
| Konservierungsmittel | 0,50 | 0,50 | 0,50 | 0,80 | 0,80 | 1,5 | 2,0 |
| pH-Stellmittel | ad pH | ad pH | ad pH | ad pH | ad pH | ad pH | ad pH |
| Wasser | ad 100 | ad 100 | ad 100 | ad 100 | ad 100 | ad 100 | ad 100 |

| | | | | | | | |
|---|---|---|---|---|---|---|---|
| ^{b)} Eumulgin B3 (INCl: Ceteareth-30; BASF) | | | | | | | |

## Patentansprüche

1. Schweißhemmendes kosmetisches Mittel, enthaltend
a) mindestens einen Stoff, ausgewählt aus der Gruppe von kosmetischen Ölen, welche bei 20 °C und 1.013 hPa flüssig sind, Riechstoffen und Wachsen,
b) Treibmittel in einer Gesamtmenge von 0 bis 99 Gew.-%, bezogen auf das Gesamtgewicht des schweißhemmenden kosmetischen Mittels, und
c) mindestens ein aus den Samen der Gattung *Adansonia* isoliertes Protein in einer Gesamtmenge von 2 bis 40 Gew.-%, bezogen auf das Gesamtgewicht des schweißhemmenden kosmetischen Mittels, wobei das Protein hydrolysiert ist,
wobei das schweißhemmende kosmetische Mittel keine Halogenide und/oder Hydroxyhalogenide von Aluminium und/oder Zirconium enthält

2. Schweißhemmendes kosmetisches Mittel nach Anspruch 1, **dadurch gekennzeichnet, dass** das mindestens eine Protein einen Calciumgehalt von 300 bis 350 mg, einen Phosphorgehalt von 1.500 bis 1.700 mg, einen Schwefelgehalt von 410 bis 450 mg, jeweils bezogen auf 1 kg des Proteins, sowie einen Kaliumgehalt von 0,3 bis 0,5 Gew.-%, bezogen auf das Gesamtgewicht des Proteins, aufweist.

3. Schweißhemmendes kosmetisches Mittel nach Anspruch 1 oder 2, **dadurch gekennzeichnet, dass** das mindestens eine Protein in einer Gesamtmenge von 2,5 bis 30 Gew.-%, insbesondere von 3 bis 20 Gew.-%, bezogen auf das Gesamtgewicht des schweißhemmenden kosmetischen Mittels, enthalten ist.

4. Schweißhemmendes kosmetisches Mittel nach einem der vorangehenden Ansprüche, **dadurch gekennzeichnet, dass** das mindestens eine Protein ein mittleres Molekulargewicht Mw von 150 bis 100.000 Da, vorzugsweise von 180 bis 50.000 Da, bevorzugt von 200 bis 10.000 Da, weiter bevorzugt von 250 bis 8.000 Da, insbesondere von 300 bis 5.000 Da, aufweist.

5. Nicht-therapeutisches kosmetisches Verfahren zur Verhinderung und/oder Reduzierung der Transpiration des Körpers, bei welchem ein schweißhemmendes kosmetisches Mittel nach einem der Ansprüche 1 bis 4 auf die Haut, insbesondere auf die Haut der Achselhöhlen, aufgetragen wird und für mindestens 1 Stunde, vorzugsweise für mindestens 2 Stunden, bevorzugt für mindestens 4 Stunden, insbesondere für mindestens 6 Stunden, auf der Haut der Achselhöhlen verbleibt.

## Claims

1. An antiperspirant cosmetic agent containing
a) at least one substance selected from the group of cosmetic oils which are liquid at 20 °C and 1,013 hPa, fragrances and waxes,
b) propellants in a total amount of 0 to 99 wt.%, based on the total weight of the antiperspirant cosmetic agent, and
c) at least one protein isolated from the seeds of the genus *Adansonia* in a total amount of 2 to 40 wt.%, based on the total weight of the antiperspirant cosmetic agent, wherein the protein is hydrolyzed,
wherein the antiperspirant cosmetic agent does not contain any aluminum and/or zirconium halides and/or hydroxy halides.

2. The antiperspirant cosmetic agent according to claim 1, **characterized in that** the at least one protein has a calcium content of 300 to 350 mg, a phosphorus content of 1,500 to 1,700 mg, a sulfur content of 410 to 450 mg, based in each case on 1 kg of the protein, and a potassium content of 0.3 to 0.5 wt.%, based on the total weight of the protein.

3. The antiperspirant cosmetic agent according to claim 1 or 2, **characterized in that** the at least one protein is contained in a total amount of 2.5 to 30 wt.%, in particular 3 to 20 wt.%, based on the total weight of the antiperspirant cosmetic agent.

4. The antiperspirant cosmetic agent according to one of the preceding claims, **characterized in that** the at least one protein has an average molecular weight Mw of 150 to 100,000 Da, preferably 180 to 50,000 Da, more preferably 200 to 10,000 Da, even more preferably 250 to 8,000 Da, in particular 300 to 5,000 Da.

5. A non-therapeutic cosmetic method for preventing and/or reducing body perspiration, in which an antiperspirant cosmetic agent according to one of claims 1 to 4 is applied to the skin, in particular to the underarm skin, and remains on the underarm skin for at least 1 hour, preferably for at least 2 hours, more preferably for at least 4 hours, in particular for at least 6 hours.

## Revendications

1. Agent cosmétique antisudoral, contenant
a) au moins une substance, choisie dans le groupe des huiles cosmétiques, qui sont liquides à 20 °C et 1,013 hPa, des substances odoriférantes et des cires,
b) un agent propulseur dans une quantité totale allant de 0 à 99 % en poids, par rapport au poids total de l'agent cosmétique antisudoral, et
c) au moins une protéine isolée à partir des graines du genre *Adansonia,* en une quantité totale allant de 2 à 40 % en poids, par rapport au poids total de l'agent cosmétique antisudoral, dans lequel la protéine est hydrolysée,
l'agent cosmétique antisudoral ne comprenant pas d'halogénure et/ou d'hydroxyhalogénure d'aluminium et/ou de zirconium.

2. Agent cosmétique antisudoral selon la revendication 1, **caractérisé en ce que** l'au moins une protéine a une teneur en calcium allant de 300 à 350 mg, une teneur en phosphore allant de 1 500 à 1 700 mg, une teneur en soufre allant de 410 à 450 mg, par rapport à 1 kg de protéine respectivement, ainsi qu'une teneur en potassium allant de 0,3 à 0,5 % en poids, par rapport au poids total de la protéine.

3. Agent cosmétique antisudoral selon la revendication 1 ou la revendication 2, **caractérisé en ce que** l'au moins une protéine est présente en une quantité totale allant de 2,5 à 30 % en poids, en particulier allant de 3 à 20 % en poids par rapport au poids total de l'agent cosmétique antisudoral.

4. Agent cosmétique antisudoral selon l'une des revendications précédentes, **caractérisé en ce que** l'au moins une protéine a une masse moléculaire moyenne Mw allant de 150 à 100 000 Da, de préférence de 180 à 50 000 Da, préférablement de 200 à 10 000 Da, de manière davantage préférée de 250 à 8 000 Da, en particulier de 300 à 5 000 Da.

5. Procédé cosmétique non thérapeutique visant à empêcher et/ou réduire la transpiration du corps, dans lequel un agent cosmétique antisudoral selon l'une des revendications 1 à 4 est appliqué sur la peau, en particulier sur la peau des aisselles et reste sur la peau des aisselles pendant au moins 1 heure, de préférence pendant au moins 2 heures, préférablement pendant au moins 4 heures, en particulier pendant au moins 6 heures.
